# EUROPÄISCHE PATENTSCHRIFT

(11) **EP 2 067 773 B1**
(45) Veröffentlichungstag und Bekanntmachung des Hinweises auf die Patenterteilung: **03.11.2010**
(21) Anmeldenummer: 08020269.0
(22) Anmeldetag: 21.11.2008
(51) Int. Cl.: C07D 229/00, C07D 471/04, C08G 18/79

(54) **Herstellung von Uretdion-Polyisocyanaten**
Production of uretdione polyisocyanates
Fabrication d'uretdiones-polyisocyanates

(30) Priorität: 04.12.2007 DE 102007058487
(43) Veröffentlichungstag der Anmeldung: 10.06.2009
(73) Patentinhaber: Bayer MaterialScience AG, 51368 Leverkusen (DE)
(72) Erfinder: Richter, Frank, Dr., 51373 Leverkusen (DE); Hecking, Andreas, 40764 Langenfeld (DE); Halpaap, Reinhard, Dr., 51519 Odenthal (DE)

(56) Entgegenhaltungen:
- WO-A-2004/005364
- DE-A1- 3 739 549
- GB-A- 821 158

## Beschreibung

Die Erfindung betrifft ein Verfahren zur Herstellung hoch uretdiongruppenhaltiger Polyisocyanate.

Uretdiongruppen aufweisende aliphatische Polyisocyanate auf Basis ggf. verzweigter, linearaliphatischer Diisocyanate zeichnen sich durch eine besonders niedrige Viskosität aus. Produkte auf Basis aromatischer, araliphatischer oder cycloaliphatischer Diisocyanate sind in der Regel hochviskose bis feste Substanzen, die u.a. als abspalterfreie, intern blockierte Vernetzer in Beschichtungssystemen eingesetzt werden können.

Eine Übersicht zur Isocyanat-Oligomerisierung wird in J. Prakt. Chem./Chem. Ztg. 1994, 336, 185--200 gegeben.

Tris(dialkylamino)phosphine (DE-A 3 030 513) ggf. in Verbindung mit Cokatalysatoren (DE-A 3 437 635) weisen eine gute Selektivität für die Bildung von Uretdiongruppen (Uretdionselektivität) auf. Ihrer technischen Einsetzbarkeit steht allerdings der schwerwiegende Makel des hohen krebserzeugenden Potenzials ihrer Phosphoroxide, z.B. Hexamethylphosphorsäuretriamid, entgegen.

DE-A 1 670 720 offenbart die Herstellung von Uretdiongruppen aufweisenden aliphatischen Polyisocyanaten, wobei als Katalysatoren tertiäre Phosphine mit mindestens einem aliphatischen Substituenten sowie Bortrifluorid und seine Addukte eingesetzt werden. Es wird darauf hingewiesen, dass nur bei niedrigen Umsätzen und Reaktionstemperaturen zwischen 50 und 80°C hohe Anteile an Uretdiongruppen im Produkt erhalten werden können, wobei gleichzeitig Isocyanat-Trimere (Isocyanurate und Iminooxadiazindione) und, insbesondere bei höherer Temperatur, auch andere Nebenprodukte wie Carbodiimide oder Uretonimine gebildet werden. Uretonimine sind ganz besonders störend, da sie bei Lagerung zur Freisetzung von monomerem Diisocyanat neigen.

Die DE-A 10254878 beschreibt die Verwendung von Phosphinen, die mindestens einen cycloaliphatischen, P-gebundenen Rest aufweisen als Katalysatoren für die NCO-Dimerisierung. Diese Katalysatoren zeichnen sich durch eine wesentlich höhere Uretdionselektivität verglichen mit anderen Trialkylphosphinen des Standes der Technik aus. Den Einsatz eines Spezialfalls dieser Phosphine, bicyclische Reste aufweisende Vertreter, für den gleichen Einsatz wird in DE 10354544 beschrieben.

Alle Phosphine weisen allerdings den gemeinsamen Nachteil der Luftempfindlichkeit auf, was ihre technische Verwendung erschwert. Auch ist ihre Uretdionselektivität stark abhängig von der Reaktionstemperatur und dem Umsatzgrad des Monomers.

DE-A 3 739 549 offenbart die katalytische NCO-Dimerisierung mit 4-Dialkylaminopyridinen, wie z.B. 4-Dimethylaminopyridin (DMAP), wobei allerdings nur im Fall spezieller cycloaliphatischer Isocyanate wie Isophorondiisocyanat (IPDI) die Uretdionbildung selektiv verläuft. Linearaliphatische Isocyanate wie Hexamethylendiisocyanat (HDI) sowie verzweigte, linearaliphatische Isocyanate wie Trimethylhexandiisocyanat (TMDI) und Methylpentandiisocyanat (MPDI) liefern mit DMAP und verwandten Verbindungen hauptsächlich stark gefärbte, heterogene Reaktionsprodukte.

Umso überraschender ist es, dass Pyridinderivate, die in 3- und 4-Position am Pyridinring durch N-Atome substituiert sind, wobei letztere beiden N-Atome mittels eines zweigliedrigen, gesättigten (sp³-hybridisierten) Kohlenstoffsegmentes verbrückt sind, äußerst wirkungsvolle Katalysatoren für die Uretdionbildung nicht nur gegenüber IPDI sind, sondern auch linearaliphatische Isocyanate wie Hexamethylendiisocyanat (HDI) in nahezu trimerfreie Polyisocyanate überführen.

Unter dem Begriff "Trimer" wird im Rahmen der vorliegenden Erfindung die Summe der durch 'Trimerisierung' aus Isocyanaten gebildeten isomeren Strukturen, Isocyanurate und Iminooxadiazindione, verstanden.

Ein weiterer Gegenstand der Erfindung ist ein Verfahren zur 'Dimerisierung' von Isocyanaten, bei dem
a) mindestens ein organisches Isocyanat,
b) ein Katalysator enthaltend mindestens ein erfindungswesentliches Pyridinderivat, der Formel 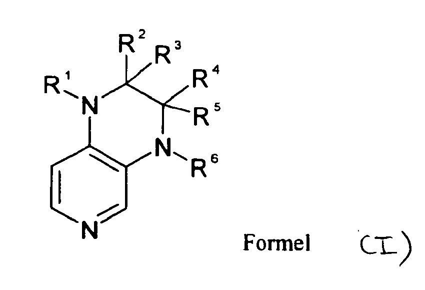 wobei
   - R¹, R⁶: unabhängig voneinander gleiche oder verschiedene, gegebenenfalls substituierte und/oder verzweigten Kohlenwasserstoffreste sind
   - R², R⁴: unabhängig voneinander Wasserstoff oder gleiche oder verschiedene, gegebenen- falls mit Heteroatomen oder funktionellen Gruppen substituierte und/oder verzweig- te Kohlenwasserstoffreste sind
   und
   - R³, R⁵: unabhängig voneinander Reste gemäß der Definition der Reste R², R⁴ sind oder zu- sammen ein cyclisches, die beiden N-Atome verbrückendes 4 bis 7 gliedriges Koh- lenstoffsegment bilden, welches durch Kohlenwasserstoffreste, Heteroatome oder funktionelle Gruppen substituiert und/oder ungesättigt sein kann.
c) optional Lösemittel und
d) optional Additive
zur Reaktion gebracht werden.

Die stereochemische Anordnung der Reste R² bis R⁵ zueinander in Formel (II) ist rein willkürlich gewählt.

Sind R², R⁴ Kohlenwasserstoffreste, so können sie sowohl linearaliphatisch, als auch cycloaliphatisch oder aromatisch sein. Sind R², R⁴ linearaliphatisch, so weisen sie bevorzugt 1 bis 12, besonders bevorzugt 1 bis 6 Kohlenstoffatome auf. Sind R², R⁴ cycloaliphatisch, so weisen sie bevorzugt 3 bis 12, besonders bevorzugt 3 bis 6 Kohlenstoffatome auf. Sind R², R⁴ aromatisch, so weisen sie bevorzugt 6 bis 20, besonders bevorzugt 6 bis 12 Kohlenstoffatome auf.

R², R⁴ können dabei durch Heteroatome oder Ethergruppen substituiert sein.

R³, R⁵ entsprechen entweder der vorstehenden Definition der Reste R², R⁴ einschließlich der Vorzugsbereiche oder bilden zusammen einen verbrückenden Rest der vorstehend genannten Art, der bevorzugt 4 bis 20, besonders bevorzugt 4 bis 12 Kohlenstoffatome aufweist und mit Heteroatomen oder funktionellen Gruppen substituiert und/oder ungesättigt sein kann.

Bevorzugte Verbindungen der Formel (I) sind solche, in denen R¹ und R⁶ unabhängig voneinander für gleiche oder verschiedene Alkylgruppen, bevorzugt ausgewählt aus der Gruppe bestehend aus Methyl, Ethyl, Propyl und Butyl stehen, R² und R⁴ unabhängig voneinander H oder gleiche oder verschiedene Alkylgruppen ausgewählt aus der Gruppe bestehend aus Methyl, Ethyl, Propyl und Butyl sowie R³ und R⁵ zusammen eine 1,3-Propylen-, 1,3-Butylen-, 2,4-Pentylen-, 1,4-Butylen-, 1,4-Pentylen-, 2,4-Hexylen-, 1,2-Cyclopentylen- oder 1,2-Cyclohexylen-Verbrückung bilden.

Mit "Propyl" bzw. "Butyl" sind jeweils alle entsprechenden isomeren Verbindungen gemeint.

Beispiele erfindungsgemäß einzusetzender 3,4-Diaminopyridine sind solche der nachfolgenden Formeln (II) bis (X) :

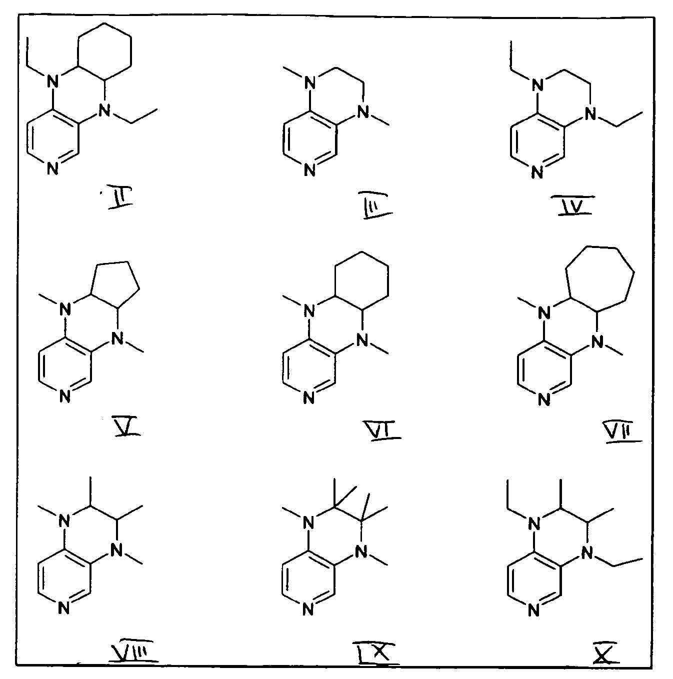

Diese können als Katalysator für die Uretdionbildung einzeln, in beliebigen Mischungen untereinander oder in Mischungen mit anderen Katalysatoren des Standes der Technik verwendet werden.

Die Menge des im erfindungsgemäßen Verfahren einzusetzenden Katalysators richtet sich in erster Linie nach dem verwendeten Isocyanat und der angestrebten Reaktionsgeschwindigkeit und liegt im Bereich 0,01 bis 5 mol-%, bezogen auf die Summe der Stoffmengen des eingesetzten Isocyanates und des Katalysators. Bevorzugt werden 0,05 bis 2 mol-% Katalysator eingesetzt.

Der Katalysator b) kann im erfindungsgemäßen Verfahren unverdünnt oder in Lösungsmitteln gelöst eingesetzt werden. Als Lösungsmittel kommen dabei eine Vielzahl von Verbindungen in Frage, beispielhaft seinen genannt: ggf. halogenierte, aliphatische oder aromatische Kohlenwasserstoffe, Alkohole, Ketone, Ester sowie Ether.

Als in a) erfindungsgemäß einzusetzende Isocyanate können prinzipiell alle bekannten, durch Phosgenierung oder nach phosgenfreien Verfahren hergestellten organischen Isocyanate einzeln oder in beliebigen Mischungen untereinander verwendet werden.

Bevorzugt ist die Verwendung von aliphatischen, cycloaliphatischen, aromatischen oder araliphatischen Di- oder Polyisocyanaten einer NCO-Funktionalität ≥ 2.

Beispielhaft genannt seien Toluylendiisocyanat (TDI), Bis(isocyanatophenyl)methan und Polyphenyl-polymethylen-polyisocyanate, wie sie durch Anilin-Formaldehyd-Kondensation und anschließende Phosgenierung hergestellt werden (MDI), Hexamethylendiisocyanat (HDI), Isophorondiisocyanat (IPDI) und Bis(isocyanatocyclohexyl)methan.

Das erfindungsgemäße Verfahren wird so geführt, dass der Umsatz der NCO-Gruppen, bevorzugt von 5 bis 90 mol-%, insbesondere 10 bis 60 mol-%, ganz besonders bevorzugt von 10 bis 50 mol-% beträgt.

Das erfindungsgemäße Verfahren wird üblicherweise im Temperaturbereich 0°C bis 150°C durchgeführt.

Um Umsätze der NCO-Gruppen gemäß der vorstehenden Bereiche zu realisieren, wird die Reaktion bei dem gewünschten Umsetzungsgrad abgebrochen.

Zum Abbruch der Reaktion nach Erreichen des gewünschten Umsetzungsgrades eignen sich eine Reihe von vorbeschriebenen Katalysatorgiften (DE-A 1670666, 1670720, 3437635) insbesondere Alkylierungsmittel, z.B. Dimethylsulfat, Toluolsulfonsäuremethylester, oder Acylierungsmittel, z.B. Säurechloride oder Säureanhydride, die mit dem Katalysator ggf. unter Temperaturerhöhung zur Reaktion gebracht werden (Variante A).

Nach der Deaktivierung der Reaktionsmischung nach Variante A kann nicht umgesetztes Monomer und/oder der deaktivierte Katalysator abgetrennt werden.

Das Verfahren kann auch ohne chemische Deaktivierung des Katalysators abgebrochen werden. Dazu wird unmittelbar nach Erreichen des gewünschten Umsatzes der aktive Katalysator aus der Reaktionsmischung abgetrennt, um eine Weiterreaktion ggf. unter Nebenproduktbildung zu unterbinden. (Variante B).

Gleichzeitig mit oder auch nach der Katalysatorabtrennung kann nicht umgesetztes Monomer aus der nach Variante B behandelten Reaktionsmischung abgetrennt werden.

Im erfindungsgemäßen Verfahren können zur Abtrennung nicht umgesetzter Monomere, des Katalysators und/oder anderer unerwünschter Bestandteile aus der Reaktionsmischung alle bekannten Separationstechniken wie z.B. Destillation, Extraktion oder Kristallisation/Filtration verwendet werden. Bevorzugt ist die Destillation, ggf. in der speziellen Ausführungsform der Dünnschichtdestillation. Selbstverständlich können auch Kombinationen zweier oder mehrerer dieser Techniken angewendet werden.

Bevorzugt wird zum Reaktionsabbruch nach Variante B der Katalysator destillativ entfernt, wobei gleichzeitig ggf. nicht umgesetztes Monomer mit entfernt wird.

Bevorzugt wird bei der Aufarbeitung einer nach Variante A oder B abgebrochenen Reaktion das enthaltene Restmonomer destillativ entfernt.

Soll das erfindungsgemäß hergestellte Polyisocyanat noch freies, nicht umgesetztes Monomer enthalten, wie es z.B. für die Weiterverarbeitung zu NCO-blockierten Produkten oder NCO-armen bzw. -freien Polyuretdionhärtern z.B. für den Pulverlackbereich von Interesse ist, so kann nach Reaktionsabbruch (Varianten A und B) auf die Monomerenabtrennung verzichtet werden.

Es ist für die Durchführung des erfindungsgemäßen Verfahrens unerheblich, ob das Verfahren ganz oder teilweise diskontinuierlich oder kontinuierlich durchgeführt wird.

Weiterhin können im erfindungsgemäßen Verfahren zu einem beliebigen Zeitpunkt, in der Polyisocyanatchemie übliche Additive und Stabilisatoren zugesetzt werden. Beispiele sind Antioxidanzien, wie z.B. sterisch gehinderte Phenole (2,6-Di-tert.butylphenol, 4-Methyl-2,6-di-tert.butylphenol), Lichtschutzmittel, wie z.B. HALS-Amine, Triazole etc., schwache Säuren oder Katalysatoren für die NCO-OH-Reaktion wie Dibutylzinndilaurat (DBTL).

Des weiteren kann es sinnvoll sein, einem nach Variante B aufgearbeiteten Produkt, geringe Mengen eines in Variante A zu verwendenden Katalysatorgiftes zuzusetzen, um die Rückspaltstabilität zu erhöhen und die Neigung zur Nebenproduktbildung, zur Verfärbung bzw. zur Weiterreaktion der freien NCO-Gruppen untereinander, z.B. bei Produktlagerung, zu unterdrücken.

Nach dem erfindungsgemäßen Verfahren hergestellte Produkte auf Basis ggf. verzweigter, linearaliphatischer Di- oder Polyisocyanate, die keine Cycloalkylsubstituenten aufweisen haben eine Viskosität < 1000 mPas/23°C. Werden cycloaliphatische, aromatische und/oder araliphatische Di- oder Polyisocyanate eingesetzt, werden hochviskose bis feste Harze erhalten (Viskosität > 10000 mPas/23°C).

In monomerenarmer Form, d.h. nach Abtrennung von nicht umgesetztem Monomer, weisen die erfindungsgemäßen Produkte einen NCO-Gehalt < 30 Gew.-%, bevorzugt < 25 Gew.-%, auf.

Die nach dem erfindungsgemäßen Verfahren hergestellten Polyisocyanate dienen als Ausgangsmaterialien zur Herstellung von z.B. Formkörpern (ggf. geschäumt), Lacken, Beschichtungsmitteln, Klebstoffen oder Zuschlagstoffen, wobei die enthaltenen freien, nicht uretdionisierten NCO-Gruppen auch ggf. blockiert sein können.

Zur Blockierung der freien, nicht uretdionisierten NCO-Gruppen eignen sich alle dem Fachmann bekannten Methoden. Als Blockierungsmittel können insbesondere Phenole (z.B. Phenol, Nonylphenol, Kresol), Oxime (z.B. Butanonoxim, Cyclohexanonoxim), Lactame (z.B. ε-Caprolactam), sekundäre Amine (z.B. Diisopropylamin), Pyrazole (z.B. Dimethylpyrazol), Imidazole, Triazole) oder Malon- und Essigsäureester verwendet werden.

Die nach dem erfindungsgemäßen Verfahren hergestellten, weitgehend nebenproduktfreien, Uretdiongruppen aufweisenden Polyisocyanate können insbesondere zur Herstellung von Ein- und Zweikomponenten-Polyurethanlacken ggf. in Mischungen mit anderen Di- oder Polyisocyanaten des Standes der Technik, wie Biuret-, Urethan-, Allophanat-, Isocyanurat-, sowie Iminooxadiazindiongruppen enthaltenden Di- oder Polyisocyanaten eingesetzt werden.

Ebenfalls besonders bevorzugt ist die Verwendung der erfindungsgemäß hergestellten Polyisocyanate auf Basis ggf. verzweigter, linearaliphatischer Isocyanate als Reaktivverdünner zur Viskositätserniedrigung höherviskoser Polyisocyanat-Harze.

Zur Umsetzung der erfindungsgemäß hergestellten Polyisocyanate zum Polyurethan können alle Verbindungen mit mindestens zwei isocyanatreaktiven Funktionalitäten einzeln oder in beliebigen Mischungen untereinander (isocyanatreaktives Bindemittel) eingesetzt werden.

Bevorzugt ist die Verwendung eines oder mehrerer, in der Polyurethanchemie an sich bekannter, isocyanatreaktiver Bindemittel wie Polyhydroxyverbindungen oder Polyamine. Als Polyhydroxyverbindungen werden besonders bevorzugt Polyester-, Polyether-, Polyacrylat- und/oder Polycarbonat-Polyole, ggf. auch unter Zusatz niedermolekularer, mehrwertiger Alkohole eingesetzt.

Das Äquivalentverhältnis zwischen nicht uretdionisierter Isocyanatgruppe, die ggf. auch blockiert sein kann, und isocyanatreaktiver Funktionalität des isocyanatreaktiven Bindemittels, wie z.B. OH-, NH- oder COOH, liegt von 0,8 bis 3, vorzugsweise 0,8 bis 2.

Möglich ist der Einsatz eines Überschusses an isocyanatreaktivem Bindemittel, da die Spaltung des Uretdionringes ggf. bei erhöhter Temperatur und/oder Katalysatorzusatz zur Freisetzung weiterer NCO-Gruppen führt, die mit dem Überschuss an isocyanatreaktiven Funktionalitäten reagieren können. Dadurch erhöht sich die Netzwerkdichte des gebildeten Polymers und dessen Eigenschaften werden vorteilhaft beeinflusst.

Für die Beschleunigung der Vernetzungsreaktion der erfindungsgemäß hergestellten Polyisocyanate mit dem isocyanatreaktiven Bindemittel können alle aus der Polyurethanchemie bekannten Katalysatoren verwendet werden. Beispielweise können Metallsalze wie Dibutylzinn-IV-dilaurat, Zinn-II-bis(2-ethylhexanoat), Wismut-III-tris(2-ethylhexanoat), Zink-II-bis(2-ethylhexanoat) oder Zinkchlorid sowie tertiäre Amine wie 1,4-Diazabicyclo(2,2,2)oktan, Triethylamin oder Benzyldimethylamin verwendet werden.

Bei der Formulierung werden das erfindungsgemäß hergestellte, ggf. blockierte Polyisocyanat, das isocyanatreaktive Bindemittel, Katalysator(en) und ggf. die üblichen Zusätze wie Pigmente, Füllstoffe, Additive, Verlaufshilfsmittel, Entschäumer und/oder Mattierungsmittel miteinander auf einem üblichen Mischaggregat wie z.B. einer Sandmühle, ggf. unter Verwendung von Lösungsmitteln, vermischt und homogenisiert.

Als Lösungsmittel geeignet sind alle an sich bekannten üblichen Lacklösemittel wie z.B. Ethyl- und Butylacetat, Ethylen- oder Propylenglykolmono-methyl-, -ethyl- oder -propyletheracetat, 2-Butanon, 4-Methyl-2-pentanon, Cyclohexanon, Toluol, Xylol, Solventnaphtha, N-Methylpyrrolidon etc.

Die Beschichtungsmittel können in Lösung oder aus der Schmelze sowie ggf. in fester Form (Pulverlacke) nach den üblichen Methoden wie z.B. Streichen, Rollen, Gießen, Spritzen, Tauchen, dem Wirbelsinterverfahren oder durch elektrostatische Sprühverfahren auf dem zu beschichtenden Gegenstand appliziert werden.

Als Substrate eignen sich sämtliche bekannten Werkstoffe, insbesondere Metalle, Holz, Kunststoffe und Keramik.

### Beispiele:

Die Ermittlung des NCO-Gehaltes der in den Beispielen und Vergleichsbeispielen beschriebenen Harze erfolgte durch Titration gemäß DIN 53 185.

Die dynamischen Viskositäten wurden bei 23°C mit dem Viskosimeter VT 550, Fa. Haake, Karlsruhe bestimmt. Durch Messungen bei unterschiedlichen Schergeschwindigkeiten wurde sichergestellt, dass das Fließverhalten der beschriebenen erfindungsgemäß hergestellten Polyisocyanate wie auch das der Vergleichsprodukte dem idealer Newtonscher Flüssigkeiten entspricht. Die Angabe der Schergeschwindigkeit kann deshalb entfallen.

Die Angaben 'mol-%' bzw. molares Verhältnis unterschiedlicher Strukturtypen zueinander' basieren auf NMR-spektroskopischen Messungen. Sie beziehen sich immer, wenn nicht anders angegeben, auf die Summe der durch die Modifizierungsreaktion (Oligomerisierung) aus den vorher freien NCO-Gruppen des zu modifizierenden Isocyanates gebildeten Strukturtypen.

¹³C-NMR-Messungen erfolgten auf den Geräten DPX 400, AVC 400 bzw. DRX 700 der Fa. Bruker, Karlsruhe, DE an ca. 50 %-igen Proben in trockenem CDCl₃ bzw. an ca. 80 %-igen Proben in D₆-DMSO (¹³C-NMR: 100 bzw 176 MHz, relaxation delay: 4 sec, mind. 2000 scans). Als Referenz für die ppm-Skala wurden geringe Mengen Tetramethylsilan im entsprechenden Lösungsmittel (δ = 0 ppm) oder das Lösungsmittel alleine (δ = 77,0 ppm (CDCl₃) bzw. δ = 43,5 ppm (D₆-DMSO)) gewählt.

Soweit nicht anders angegeben, wurden die Reaktionen mit frisch entgastem HDI oder IPDI als Edukt durchgeführt. Die Bezeichnung frisch entgast' bedeutet dabei, dass das eingesetzte Diisocyanat unmittelbar vor der katalytischen Umsetzung durch mindestens 30-minütiges Rühren im Vakuum (< 1 mbar) von gelösten Gasen befreit und anschließend mit Stickstoff belüftet wurde.

Alle Reaktionen wurden unter einer Atmosphäre von trockenem Stickstoff durchgeführt.

Die verwendeten Katalysatoren wurden nach literaturbekannten Methoden hergestellt (Synthesis 2007, No. 8, 1185-1196). In den erfindungsgemäßen Beispielen werden die Katalysatoren (II) und (III) gemäß vorstehender Formeln der Beschreibung verwendet.

### Beispiele 1 und 2, erfindungsgemäß sowie 3 und 4, Vergleichsbeispiele

Jeweils 10 g frisch entgastes HDI (Bsp. 1 und 3) bzw. IPDI (Bsp. 2 und 4) wurden in mit Septen verschlossenen Glasgefäßen unter Stickstoff in Gegenwart von jeweils 2-mol-% Katalysator (II) (Bsp. 1 und 2) bzw. N,N-Dimethylaminopyridin (DMAP) (Bsp. 3 und 4) bei 23°C mit einem Magnetrührkem gerührt, wobei in regelmäßigen Abständen der Fortgang der Reaktion durch Messung des Brechungsindex' (bei 20°C und der Frequenz des Lichtes der D-Linie des Natriumspektrums: n_{D}²⁰) der Reaktionsmischung überprüft wurde, vgl. Tabelle 1. Nach 135 Minuten wurde mittels NMR-Spektroskopie analysiert. Nach 24 h waren die Reaktionsmischungen aus Beispiel 1 und 2 hochviskos und nicht mehr mittels Magnetrührkern rührbar, während die aus den Beispielen 3 und 4 noch gut rührbar waren. Letzterer Umstand belegt die deutlich höhere Aktivität des erfindungsgemäßen Katalysators gegenüber dem des Standes der Technik (DMAP).

| | Beispiel 1 | Beispiel 2 | Beispiel 3 | Beispiel 4 |
|---|---|---|---|---|
| | erfindungsgemäß | erfindungsgemäß | Vergleich | Vergleich |
| Katalysator | (II) | (II) | DMAP | DMAP |
| Isocyanat | HDI | IPDI | HDI | IPDI |
| n_{D}²⁰ bei t₀* | 1,4581 | 1,4846 | 1,4580 | 1,4859 |
| t = 15 min | 1,4595 | 1,4851 | 1,4593 | 1,4862 |
| Farbe: | schwach gelb | farblos | tiefrot-orange | schwach gelb |
| t = 75 min | 1,4653 | 1,4897 | 1,4605 | 1,4866 |
| t= 135 min | 1,4711 | 1,4968 | 1,4701 | 1,4906 |
| molares Verhältnis Uretdione : 'Trimere' | 97:3 | kein Isocyanurat nachweisbar | 2:1 | 98:2 |

| | | | | |
|---|---|---|---|---|
| * Reaktionsbeginn; Katalysator vollst. gelöst | | | | |

### Beispiel 5, erfindungsgemäß

Katalysator: (II) (0,24 mol%, bezogen auf HDI); Reaktionstemperatur: 40°C

In einem doppelwandigen, durch einen externen Kreislauf auf 40°C temperierten Planschliffgefäß mit Rührer, an eine Inertgasanlage (Stickstoff/Vakuum) angeschlossenem, Rückflusskühler und Thermometer wurden 400 ml HDI vorgelegt und entgast. Nach Belüften mit Stickstoff wurden 1,5 g Katalysator II zugegeben und die in Tab. 2 angegebene Zeit bei 40°C gerührt. Der Brechungsindex der Mischung (n_{D}²⁰) stieg dabei auf 1,4760. Anschließend wurde das Reaktionsgemisch ohne vorherige Desaktivierung des Katalysators aufgearbeitet. Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 mbar, VV-Temperatur: 140°C, HV-Temp.: 150°C, Destillationsdauer: 1 h), wobei nicht umgesetztes Monomer gemeinsam mit dem aktiven Katalysator als Destillat und das Uretdiongruppen enthaltende Polyisocyanatharz als Sumpfprodukt separiert wurden (Startdurchlauf: Beispiel 5-0)

Das den aktiven Katalysator enthaltende Destillat wurde in einer zweiten Planschliff-Rührapparatur, die identisch zur ersten aufgebaut ist, gesammelt und unmittelbar nach Destillationsende mit frisch entgastem HDI auf die Ausgangsmenge (400 ml) aufgefüllt. Anschließend wurde erneut die in Tab. 2 angegebene Zeit bei 40°C gerührt und destillativ aufgearbeitet wie oben beschrieben (Beispiel 5-A). Diese Verfahrensweise wurde insgesamt drei mal wiederholt (bis Versuch 5-C).

Verfährt man analog mit DMAP an Stelle von (II), erhält man bereits im ersten Destillationsdurchlauf kein brauchbares Polyisocyanatharz, sondern nur hochviskoses, stark verfärbtes, inhomogenes Material in schlechten Ausbeuten, das weder als Polyisocyanat an sich noch zur Weiterverarbeitung, beispielsweise in Pulverlackvernetzer, geeignet ist. Das erhaltene Destillat ist nahezu inaktiv.

**Tabelle 2: Katalysator: (II) (0,24 mol-%, bezogen auf HDI); Reaktionstemperatur: 40°C, semi-kontinuierliche Reaktionsführung**

| Bsp. | Reaktionszeit | Harzausbeute | NCO-Gehalt | Viskosität |
|---|---|---|---|---|
| 5- | [hh:mm] | [g] | [%] | [mPas] bei 23°C |
| 0 | 24:00 | 198 | 19,7 | 100 |
| A | 22:55 | 161 | 20,7 | 70 |
| B | 23:15 | 124 | 21,3 | 60 |
| C | 25:50 | 99 | 21,7 | 50 |

### Beispiel 6, erfindungsgemäß

Katalysator: (III) (0,2 mol-%, bezogen auf HDI); Reaktionstemperatur: 40°C

Es wurde analog Beispiel 5 verfahren, mit dem Unterschied, dass 450 ml HDI und 0,9 g Katalysator der Formel III verwendet wurden.

**Tabelle 3: Katalysator: (III) (0,2 mol%, bezogen auf HDI); Reaktionstemperatur: 40°C, semi-kontinuierliche Reaktionsführung**

| Bsp. | Reaktionszeit | Harzausbeute | NCO-Gehalt | Viskosität |
|---|---|---|---|---|
| 6- | [hh:mm] | [g] | [%] | [mPas] bei 23°C |
| 0 | 24:00 | 140 | 21,7 | 60 |
| A | 22:00 | 80 | 23,0 | 50 |
| B | 23:30 | 40 | 23,2 | 53 |
| C | 25:50 | 28 | 23,3 | 50 |

Die strukturelle Zusammensetzung der Harze aus den Beispielen 5 und 6 ist identisch. Es handelt sich um praktisch reine HDI-Uretdione, > 95 mol-% Uretdionstrukturen, neben Isocyanurat- und Iminooxadiazindionstrukturen, letztere summarisch < 5%.

## Patentansprüche

1. Verfahren zur 'Dimerisierung' von Isocyanaten, bei dem
a) mindestens ein organisches Isocyanat,
b) ein Katalysator enthaltend mindestens ein Pyridinderivat entsprechend der
Formel (I)
c) optional Lösemittel und
d) optional Additive
zur Reaktion gebracht werden. 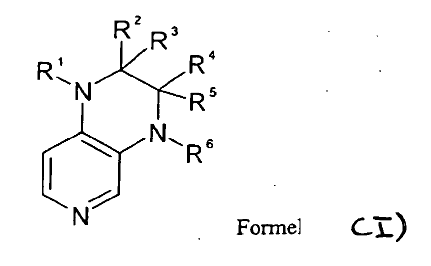 wobei
R¹, R⁶ unabhängig voneinander gleiche oder verschiedene, gegebenenfalls verzweigte Kohlenwasserstoffreste sind
R², R⁴ unabhängig voneinander Wasserstoff oder gleiche oder verschiedene, gegebenen- falls mit Heteroatomen oder Ethergruppen substituierte und/oder ver- zweigte Kohlenwasserstoffreste sind und
R³, R⁵ unabhängig voneinander Reste gemäß der Definition der Reste R², R⁴ sind oder zu- sammen ein cyclisches, die beiden N-Atome verbrückendes 4 bis 7 gliedriges Koh- lenstoffsegment bilden, welches durch Kohlenwasserstoffreste, Heteroatome oder Ethergruppen substituiert und/oder ungesättigt sein kann.

2. Verfahren gemäß Anspruch 1, **dadurch gekennzeichnet, dass** in Formel (I)
R¹ und R⁶ unabhängig voneinander für gleiche oder verschiedene Alkylgruppen, bevorzugt ausgewählt aus der Gruppe bestehend aus Methyl, Ethyl, Propyl und Butyl stehen,
R² und R⁴ unabhängig voneinander H oder gleiche oder verschiedene Alkylgruppen ausgewählt aus der Gruppe bestehend aus Methyl, Ethyl, Propyl und Butyl sowie
R³ und R⁵ zusammen eine 1,3-Propylen-, 1,3-Butylen-, 2,4-Pentylen-, 1,4-Butylen-, 1,4-Pentylen-, 2,4-Hexylen-, 1,2-Cyclopentylen- oder 1,2-Cyclohexylen-Verbrückung bilden.

3. Verfahren gemäß einem der Ansprüche 1 oder 2, **dadurch gekennzeichnet, dass** die eingesetzten Pyridine einer der Formeln (II) bis (X) entsprechen

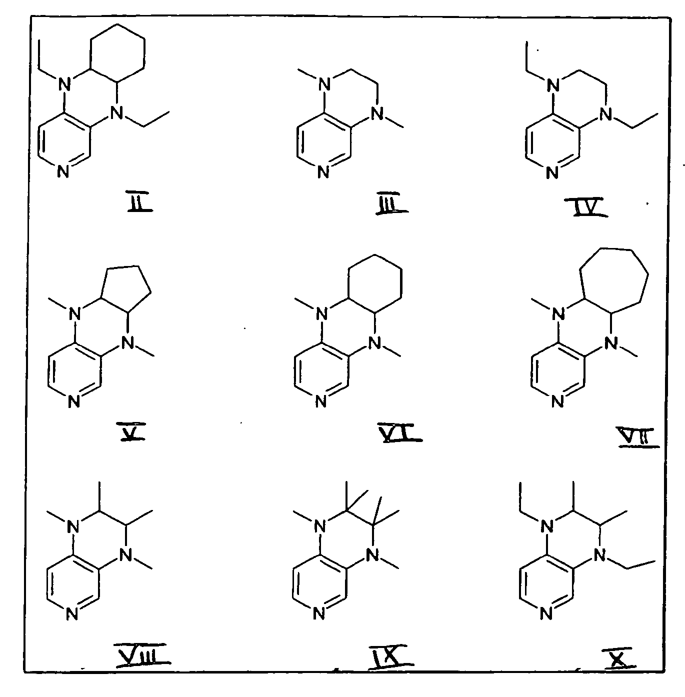

## Claims

1. Process for the "dimerization" of isocyanates, in which
a) at least one organic isocyanate,
b) a catalyst containing at least one pyridine derivative corresponding to the formula (I) 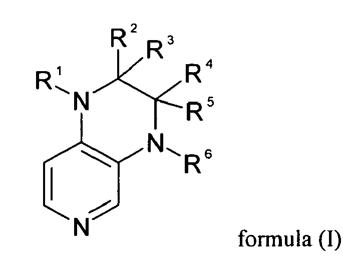 where
R¹, R⁶ are, independently of one another, identical or different, optionally branched hydrocarbon radicals,
R², R⁴ are, independently of one another, hydrogen or identical or different hydrocarbon radicals which may be substituted by heteroatoms or ether groups and/or be branched and
R³, R⁵ are, independently of one another, radicals having the definition of the radicals R², R⁴ or together form a cyclic, 4- to 7-membered hydrocarbon segment which bridges the two N atoms and may be substituted by hydrocarbon radicals, heteroatoms or ether groups and/or be unsaturated,
c) optionally solvents and
d) optionally additives
are reacted.

2. Process according to Claim 1, **characterized in that**, in formula (I)
R¹ and R⁶ are, independently of one another, identical or different alkyl groups which are preferably selected from the group consisting of methyl, ethyl, propyl and butyl,
R² and R⁴ are, independently of one another, H or identical or different alkyl groups selected from the group consisting of methyl, ethyl, propyl and butyl and
R³ and R⁵ together form a 1,3-propylene, 1,3-butylene, 2,4-pentylene, 1,4-butylene, 1,4-pentylene, 2,4-hexylene, 1,2-cyclopentylene or 1,2-cyclohexylene bridge.

3. Process according to either of Claims 1 and 2, **characterized in that** the pyridines used correspond to one of the formulae (II) to (X)

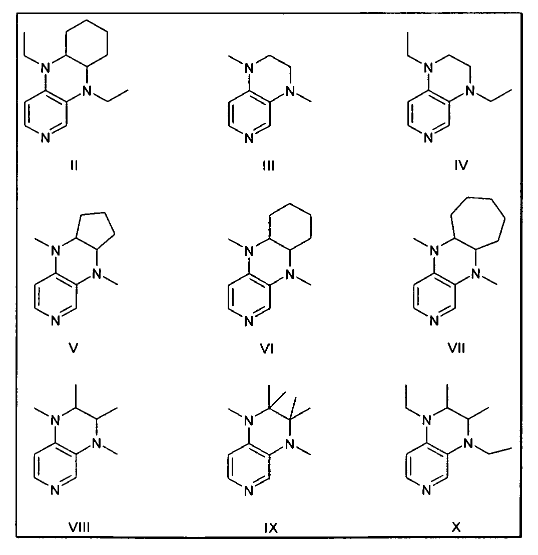

## Revendications

1. Procédé pour la « dimérisation » d'isocyanates, dans lequel on fait réagir
a) au moins un isocyanate organique,
b) un catalyseur contenant au moins un dérivé de pyridine correspondant à la formule (I) 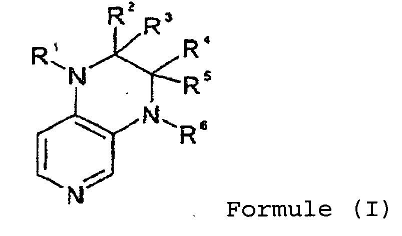 dans laquelle
R¹, R⁶ représentent, indépendamment l'un de l'autre, des radicaux hydrocarbonés éventuellement ramifiés, identiques ou différents,
R², R⁴ représentent, indépendamment l'un de l'autre, un atome d'hydrogène ou des radicaux hydrocarbonés éventuellement substitués par des hétéroatomes ou des groupes éther et/ou ramifiés, identiques ou différents et
R³, R⁵ sont indépendamment l'un de l'autre des radicaux selon la définition des radicaux R², R⁴ ou forment ensemble un segment carboné cyclique à 4-7 chaînons, pontant les deux atomes d'azote, qui peut être insaturé et/ou substitué par des radicaux hydrocarbonés, des hétéroatomes ou des groupes éther,
c) en option un solvant et
d) en option des additifs.

2. Procédé selon la revendication 1, **caractérisé en ce que** dans la formule (I)
R¹ et R⁶ représentent, indépendamment l'un de l'autre, des groupes alkyle identiques ou différents, choisis de préférence dans l'ensemble constitué par les groupes méthyle, éthyle, propyle et butyle,
R² et R⁴ représentent, indépendamment l'un de l'autre, H ou des groupes alkyle identiques ou différents, choisis dans l'ensemble constitué par les groupes méthyle, éthyle, propyle et butyle, ainsi que
R³ et R⁵ forment ensemble un pont 1,3-propylène, 1,3- butylène, 2,4-pentylène, 1,4-butylène, 1,4- pentylène, 2,4-hexylène, 1,2-cyclopentylène ou 1,2-cyclohexylène.

3. Procédé selon la revendication 1 ou 2, **caractérisé en ce que** les pyridines utilisées correspondent à l'une des formules (II) à (X)
